Europäisches Patentamt

European Patent Office

Office européen des brevets

(19)

(11) Publication number: **0 134 085**
A1

## (12) EUROPEAN PATENT APPLICATION

(21) Application number: 84304581.6

(22) Date of filing: **04.07.84**

(51) Int. Cl.⁴: **C 12 N 15/00**, C 07 H 21/04,
C 07 K 7/10, C 07 K 13/00,
C 12 P 21/02

(30) Priority: **05.07.83 US 510935**

(43) Date of publication of application: **13.03.85**
**Bulletin 85/11**

(84) Designated Contracting States: **AT BE CH DE FR GB IT
LI LU NL SE**

(71) Applicant: **THE SALK INSTITUTE FOR BIOLOGICAL
STUDIES, 10010 North Torrey Pines Road, La Jolla
California 92038 (US)**

(72) Inventor: **Gubler, Ulrich Andreas, 4 Club Street
Montclair, New Jersey 07042 (US)**
Inventor: **Ling, Nicholas Chai-Kwan, 5324 Bloch Street
San Diego, California 92122 (US)**

(74) Representative: **Lawrence, Malcolm Graham et al,
BROOKES & MARTIN High Holborn House 52/54 High
Holborn, London WC1V 6SE (GB)**

(54) DNA encoding a GRF precursor.

(57) mRNAs encoding for GRF sequences are identified in a pancreas tumor and sequenced. From the sequence, it is learned that hpGRF is initially a portion of a longer precursor peptide that is subsequently processed into hpGRF. cDNA made from such mRNA is transformed into a host cell which produces a GRF precursor. In vitro or in vivo processing can be used to obtain GRF from the precursor.

EP 0 134 085 A1

## DNA ENCODING A GRF PRECURSOR

The present invention relates to a novel peptide, designated preproGRF, which is an unprocessed precursor of human growth hormone releasing factor (GRF), DNA which encodes preproGRF and the production of preproGRF and GRF using recombinant DNA technology.

Several important hormones are produced in the mammalian hypothalamus and in the anterior lobe of the pituitary gland, one important hormone being growth hormone (GH) which promotes mammalian growth. It has been established that release of growth hormone by the pituitary is subject to regulations by a hypothalamic peptide. It has been known for some time that a hypothalamic peptide, somatostatin, inhibits release of GH. The concept has been well established that a substance produced in the hypothalamus, referred to as GRF or somatocrinin, is the counterpart of somatostatin, promoting the release of GH from the pituitary.

Although GRF is generally associated with the hypothalamus, it may be produced by other cells, such as pancreatic tumor cells. In fact, the first peptide having GRF activity that was fully characterized was obtained from a human pancreatic islet tumor, (Guillemin et al. Science 218 pp. 585-587 (1982)). It is believed that human pancreatic GRF is identical to human hypothalamic GRF, although this has not been conclusively shown. In any case, pancreatic GRF is highly effective in promoting release of growth hormone, and in view of its potential for regulating human growth, it would be highly desirable to be able to obtain human pancreatic GRF in substantial quantities.

The GRF characterized by Guillemin et al. has 44 amino acid residues and is amidated at its carboxyl end. A form of GRF having only 40 amino acid residues has also been reported, and fragments of GRF having as few as about 27 amino acid residues have been found to be biologically active, although less effective than the 44 amino acid residue peptide. The full 44 amino acid residue pancreatic GRF peptide has been synthesized chemically, and synthetic GRF, synthetic GRF fragments, and synthetic analogs thereof represent a potential source of highly potent regulatory substances; however, chemical synthesis of such long peptide chains is quite expensive.

Recombinant DNA techniques by which human GRF DNA is introduced into a cell to express GRF is believed to represent the best hope of inexpensively producing GRF. One method of obtaining DNA which encodes GRF is to read the genetic code in reverse and synthesize an oligodeoxynucleotide which should encode the GRF amino acid sequence. Such synthetic "genes" or DNA have indeed been designed. Attempts to introduce such synthetic "genes" into cells have not yet resulted in transformants which produce significant amounts of GRF.

Superior results in gene expression obtained using recombinant DNA technology are frequently obtained if DNA isolated from natural sources is used instead of oligodeoxynucleotides synthesized according to a nucleotide sequence deduced from the amino acid sequence. One reason for this may be that, although the genetic code is redundant in that several codons (a defined sequence of three successive nucleotides) generally encode a single amino acid, the natural nucleotide sequence may be superior to a random selection of apparently appropriate codons. Furthermore, it is frequently observed that a natural gene does not only encode the observed peptide, but rather encodes a precursor which contains the putative signal and other cryptic and coding sequences which undergo and/or direct obligate processing steps necessary for the production of the mature and active peptide.

This invention is directed to the isolation of the mRNA encoding GRF and its application to produce GRF through recombinant DNA techniques. As described more fully hereinafter, mRNA has been isolated encoding precursors of GRF which include the entire 44 amino acid residue sequence of human pancreatic GRF as well as flanking sequences at the carboxyl-terminus and amino-terminus of GRF.

Starting with RNA obtained from a human GRF-producing pancreatic tumor, cDNA was generated by reverse transcription, and cDNA clones encoding GRF precursors were identified from a cDNA library. Sequencing of these cDNA clones disclosed that they encoded two closely related precursors of human GRF. Each GRF precursor includes the entire 44 amino acid sequence of human pancreatic GRF as well as processing signals for directing conversion of the precursors to GRF, including amidation of its carboxyl-terminus. The cDNA clones have been used to transform cells and have been shown to direct synthesis of the GRF precursors in the transformed cells. Where processing enzymes are present in the transformed cells, the precursor is converted to GRF.

In accordance with the present invention, the search for the human growth hormone releasing factor preprohormone has led to the isolation and sequencing of genes that encode two precursors of human panceatic growth hormone releasing factor (hpGRF). Human pancreatic hormone releasing factor is a forty-four amino acid residue peptide, and is designated hpGRF-44. Based upon biological, immunological and physico-chemical evidence, hpGRF-44 is identical to human hypothalamic growth hormone releasing factor (hhGRF). One of the two cDNAs encodes a precursor having 107 amino acid residues, designated preproGRF-107, and the other encodes a precursor having 108 amino acid residues, designated preproGRF-108. The preproGRFs each contain the entire amino acid sequence of hpGRF in addition to peptide fragments linked to both the amino terminus and carboxyl terminus.  Each terminal peptide contains basic processing sites which direct their removal by enzymatic action.  Amidation of the carboxyl-terminus of mature GRF is directed by the Gly-Arg sequence in the carboxyl-terminal peptide.

Isolation of messenger RNA (mRNA) from a human pancreatic tumor which has very high human pancreatic growth hormone releasing factor activity is first accomplished, and then cDNA is synthesized from the mRNA by reverse transcription.  The cDNA was annealed to plasmids which were transformed into E. coli cells, and the cells were cultured as individual colonies, creating a cDNA library.  From the cDNA library, colonies of cells transformed with cDNA encoding peptides that contain hpGRF sequences were selected by hybridization screening using oligodeoxynucleotides synthesized to correspond to portions of the GRF amino acid sequence. The cDNA of the hybridization-positive clones were excised from the plasmid DNA and sequenced, and the nucleotide sequence revealed that the GRF amino acid sequence is derived from a portion of a larger precursor protein that is designated preproGRF.  The isolated cDNAs also include noncoding segments at their 3' and 5' ends.

The isolated cDNAs, when appropriately inserted into controlling DNA segments, direct synthesis of preproGRF in transformed cells.  From transformed cell lines that lack the preproGRF processing enzymes, preproGRF-containing polypeptides are isolated and purified.  Cell lines having the preproGRF processing enzymes, when transformed with hybrid genes containing the foreign preproGRF cDNA, produce additional GRF.  The invention will now be described in greater detail by way of example.

Isolation and sequencing of preproGRF-encoding mRNA is aided by the availability of a pancreatic tumor described in Guillemin et al. supra, having high intrinsic pancreatic growth hormone releasing activity.

3 ug Poly($A^+$)-RNA are obtained from 15 g of the tumor (stored in liquid nitrogen or at -80°C) using the guanidine thiocyanate procedure of Chirgwin et al., Biochemistry 18 5294-5294 (1979) and chromatography on oligo dT-cellulose, Norgard et al., J. Biol. Chem. 225, 7665-7672 (1980). From the 3 ug of Poly ($A^+$)-RNA, 1 ug of double-stranded cDNA is synthesized using a modification of Gubler et al. (manuscript in preparation) of the Okayama et al. method, Mol. Cell Biol. 2 161-170 (1982).

The cDNA is used to construct a cDNA library. Using a modification of the method of Peacock et al., Biochem. Biophys. Acta. 655, 243-250 (1981). The cDNA is tailed with poly dGTP and annealed to EcoRV-cut pBR322 plasmid tailed with poly dCTP. The chimeric plasmids are then transformed into E. coli RR1, and the transformed cells are plated, providing 300,000 independent transformants.

The colonies are then screened to locate colonies containing cDNA which include nucleotide sequences corresponding to the predicted nucleotide sequence of fractions of genes that encode hpGRF sequences. Oligodeoxynucleotides are synthesized which are predicted to be complementary to a segment of the natural GRF gene, and these oligodeoxynucleotides are used as hybridization probes for colony screening. Two mixed nucleotide probe pools are synthesized by the solid phase phosphotriester method of Miyoshi et al., Nucleic Acids Res. 8, 5507-5517 (1980) and labeled at their 5' ends with /$^{32}$P-ATP according to the method of Maxam et al., in Methods in Enzymology, eds. Grossman et al. Vol. 65, pp. 499-560, Academic Press, New York (1980). One of the mixed oligodeoxynucleotide probes, designated probe A, is 20 bases long and corresponds to amino acid residues 1-7 of the hpGRF sequence:

```
      1                                  7
  H-Tyr-Ala-Asp-  Ala-Ile-    Phe-Thr
  5'-ATG CGN CTG/A CGN TAT/G/A AAA TG -3'.
```

The other mixed probe B is 14 bases long and corresponds to amino acid residues 35-39 of hGRF.

```
    35                              39
  Asn-  Gln-  Glu-  Arg-           Gly
  5'-TTA/G GTT/C CTT/C GCN or TCT/C CC -3'
```

The tetradecamer probe B is initially used for colony screening by the method of Hanahan et al. Gene 10, 63-67 (1978). Hybridizations are performed in a buffer comprised of 0.75 sodium chloride, 0.075 M sodium citrate, 0.1% sodium dodecylsulfate, partially hydrolyzed yeast RNA (100 ug/ml), 0.2% bovine serum albumin(BSA), 0.2% polyvinylpyrrolidone and 0.2% ficoll at 30°C for 2 hr using 1 pmole of labeled probe per ml. The filters are quickly rinsed in a solution of 0.3 M sodium chloride, 0.03 M citrate, at room temperature, and following a high stringency wash in a solution of 0.6 M sodium chloride, 0.06 M sodium citrate for 60 min. at 40°C, the filters are exposed to X-ray film overnight. High and low density screening of 11,000 cDNA-clones with probe B reveals that the inserted DNA fragments of nine of these clones hybridize to probe B. Southern blot analysis shows that the inserts of seven of these nine recombinants also hybridize to probe A.

The plasmid DNAs of the transformants that hybridize to both probe A and probe B are linearized with Hind III and subjected to partial sequencing by the chain termination method of Smith, Methods of Enzymology 65 pp.560-580, using probe B as a primer. Four clones encoding hpGRF sequences are thereby identified of which clone no. 21 contains the largest cDNA insert (520 bp).

The cDNA insert of clone no. 21 is fully sequenced by the method of Maxam et al. supra, and its sequence is shown in the Table below.

:

page 6a follows

TABLE

```
                                                                    50
Clone  8:  TGGGAACGCCAGGCGGCTGCCAGAGCAAACACCCAGCCCAGGGCCCCTGGATTTGAGCAGTGCCT
Clone 21:                                          AGGGCCCCTGGATTTGAGCAGTGCCT


                                                 100
CGGAGCAGAGGGATATCTGCCGCATCAGGTGCCACCCCGGGTGAAGG ATG CCA CTC TGG GTG TTC
CGGAGCAGAGGGATATCTGCCGCATCAGGTGCCACCCCGGGTGAAGG ATG CCA CTC TGG GTG TTC
          preproGRF-108                          Met Pro Leu Trp Val Phe
          preproGRF-107                          Met Pro Leu Trp Val Phe


                             150
TTC TTT GTG ATC CTC ACC CTC AGC AAC AGC TCC CAC TGC TCC CCA CCT CCC CCT
TTC TTT GTG ATC CTC ACC CTC AGC AAC AGC TCC CAC TGC TCC CCA CCT CCC CCT
Phe Phe Val Ile Leu Thr Leu Ser Asn Ser Ser His Cys Ser Pro Pro Pro Pro
Phe Phe Val Ile Leu Thr Leu Ser Asn Ser Ser His Cys Ser Pro Pro Pro Pro
          10                                              20


                  200
TTG ACC CTC AGG ATG CGG CGG TAT GCA GAT GCC ATC TTC ACC AAC AGC TAC CGG
TTG ACC CTC AGG ATG CGG CGG TAT GCA GAT GCC ATC TTC ACC AAC AGC TAC CGG
Leu Thr Leu Arg Met Arg Arg Tyr Ala Asp Ala Ile Phe Thr Asn Ser Tyr Arg
Leu Thr Leu Arg Met Arg Arg Tyr Ala Asp Ala Ile Phe Thr Asn Ser Tyr Arg
                  30                                                40


              250
AAG GTG CTG GGC CAG CTG TCC GCC CGC AAG CTG CTC CAG GAC ATC ATG AGC AGG
AAG GTG CTG GGC CAG CTG TCC GCC CGC AAG CTG CTC CAG GAC ATC ATG AGC AGG
Lys Val Leu Gly Gln Leu Ser Ala Arg Lys Leu Leu Gln Asp Ile Met Ser Arg
Lys Val Leu Gly Gln Leu Ser Ala Arg Lys Leu Leu Gln Asp Ile Met Ser Arg
                               50                                    60


         300
CAG CAG GGA GAG AGC AAC CAA GAG CGA GGA GCA AGG GCA CGG CTT GGT CGT CAG
CAG CAG GGA GAG AGC AAC CAA GAG CGA GGA GCA AGG GCA CGG CTT GGT CGT CAG
Gln Gln Gly Glu Ser Asn Gln Glu Arg Gly Ala Arg Ala Arg Leu Gly Arg Gln
Gln Gln Gly Glu Ser Asn Gln Glu Arg Gly Ala Arg Ala Arg Leu Gly Arg Gln
                               70                              *


   350                                                             400
GTA GAC AGC ATG TGG GCA GAA CAA AAG CAA ATG GAA TTG GAG AGC ATC CTG GTG
GTA GAC AGC ATG TGG GCA GAA CAA AAG CAA ATG GAA TTG GAG AGC ATC CTG GTG
Val Asp Ser Met Trp Ala Glu Gln Lys Gln Met Glu Leu Glu Ser Ile Leu Val
Val Asp Ser Met Trp Ala Glu Gln Lys Gln Met Glu Leu Glu Ser Ile Leu Val
   80                               90


                                           450
GCC CTG CTG CAG AAG CAC AGC AGG AAC TCC CAG GGATGAAGATTCCTCCTGTGACCCGGG
GCC CTG CTG CAG AAG CAC     AGG AAC TCC CAG GGATGAAGATTCCTCCTGTGACCCGGG
Ala Leu Leu Gln Lys His Ser Arg Asn Ser Gln Gly
Ala Leu Leu Gln Lys His     Arg Asn Ser Gln Gly
              100


                                  500
CTACCTGTAGCCAAAATGCAACTGGATCCAGTTAATCCTCTCATTTCTGACCCACTTTTTCCTTTGAAAAT
CTACCTGTAGCCAAAATGCAACTGGATCCAGTTAATCCTCTCATTTCTGACCCACTTTTTCCTTTGAAAAT


        550                   570
ACAATAAAATTCCCCCATACCGGTGTGCATTTAAATGTTAAAAAAAAAAAAAAAAA
ACAATAAAATTCCCCCATACCGGTGTGCATTTAAA  AAAAAAAAAAAAAAAAA
```

The nucleotide sequence of clone no. 21 indicates that it encodes a polypeptide which contains the entire amino acid sequence of hpGRF-44, previously characterized by protein microsequencing. In the hpGRF reading frame, the presumed initiator and terminator codons are located 31 and 32 codons upstream and downstream, respectively, from the hpGRF-44 coding sequence. Therefore, this cDNA encodes a preproGRF that is 107 amino acids long and has the typical characteristics of a preprohormone. (Steiner et al., Annals of New York Acad. of Sci., 243:1-16 (1980)).

To confirm that the amino acid sequence of preproGRF that is deduced from the sequenced cDNA fragment in fact corresponds to the actual gene product and is not an artifact of reverse transcription, mRNA obtained from the pancreatic tumor cell is used to generate translation products, and the molecular weight of the translation product is compared with the molecular weight of the predicted preproGRF product. Translation of hpGRF-44 mRNA is carried out in the cell-free rabbit reticulocyte system. The products are immunoprecipitated with a specific antibody raised against synthetically produced GRF and then are separated by electrophoresis on a polyacrylamide gel containing sodium dodecyl sulfate. The largest translation product runs as a doublet with a molecular weight of about 13,000 that agrees well with the size of preproGRF-107 predicted from the nucleotide sequence of clone no. 21 (MW 12,361). A smaller translation product also runs as a doublet (MW 8000) and, like the larger product, is reactive with GRF-specific antibody. The low molecular weight product may represent premature translational terminations or a low level of processing of the larger peptide. The reason for the appearance of doublet bands is unclear. These results demonstrate that the nucleotide sequence is correct and that the cDNA fragment of clone 21 contains the entire preproGRF-44 coding region.

Having confirmed that clone no. 21 contains the entire coding sequence for preproGRF-44, it is now possible to use the recombinant segment to locate hpGRF-44 messenger RNA from poly $(A^+)$-RNA. Using nick-translated plasmid DNA from clone no. 21 as a hybridization probe for Northern blot analysis of poly $(A^+)$-RNA, a prominent band corresponding to a messenger RNA of approximately 820 nucleotides is detected. Because recombinant no. 21 contains a complete 3'-noncoding region and the poly($A^+$) tract, it appears that the 520bp cDNA segment transformed into clone no. 21 does not contain the entire 5' noncoding region of the preproGRF message.

Having ascertained that clone no. 21 contains an inserted DNA gene segment corresponding to the entire GRF-44 coding region, the cDNA from this clone provides longer and more specific hybridization probes useful for locating clones containing similar DNA segments. The original cDNA library is rescreened with a probe derived from the 5'-end of the cDNA insert of clone no. 21 (nucleotides no. 82-149), with respect to the Table.

One clone, no. 8, contains a DNA insert segment 40 nucleotides longer at the 5' end, an additional trinucleotide in the coding region, and an additional tetranucleotide in the 3'-non-coding region. As can be seen in the Table, the trinucleotide encodes an additional serine, residue 103, of the 108 amino acid residue peptide, preproGRF-108. Of four additional clones analyzed, three contain the nucleotide sequence encoding preproGRF-108, and one contains the sequence encoding preproGRF-107, confirming the presence of at least two different hpGRF mRNA's.

Indications are that the DNA segment in clone 8 is a substantially complete preproGRF cDNA, representing reverse transcription of substantially all of a GRF-44 messenger RNA. Further indications are that a minor portion of hpGRF mRNA may be about 80 nucleotides longer.

Having established the primary structures of two cDNAs encoding preproGRF 107 and 108, some observations are made about its structure. The first 20 amino acid residues (underlined in the Table) of preproGRF 107 and of preproGRF 108 constitute the putative hydrophobic signal peptide that is joined to proGRF, 87-88 amino acids in length. ProGRF 87 and proGRF 88 contain processing sites (boxed in the Table) for enzymatic generation of hpGRF-44, i.e., two arginine residues (30-31) preceding the amino-terminus and a single arginine residue (77) immediately following the carboxyl terminus. The sequence Gly-Arg (76-77) immediately following the carboxyl-terminus leucine of hpGRF-44 is consistent with a signal for amidation of the mature product.

The cDNA segments obtained from the hybridization-positive clones of the cDNA library are useful for introduction into either eukaryotic or prokaryotic host cells for the purpose of GRF expression. Plasmid DNA is obtained from each of clones 21 and 8. The plasmid is cut with Hind III endonuclease to expose the 5' end of the inserted cDNA fragment. The 5' sequences of preproGRF may be digested with Bal 31 exonuclease in order to generate a series of DNA segments which include variable lengths of amino terminal sequences for use in a variety of expression vectors. For example, for use in /gt11 or pCQV2 it will be necessary to remove all sequences 5' to the initiation codon for preproGRF. Chemically synthesized oligonucleotides will be then added to 5' end of the digested DNA segment according to the specification of the vectors in order to optimize the production of either a hybrid or fusion gene product for maximal levels of expression of pure preproGRF, proGRF or the GRF proteins. Linkers will be annealed to the 3' terminus (after subsequent recloning of the digested DNA segment), and the final engineered fragments can be digested with appropriate endonucleases and isolated from the larger plasmid DNA by polyacrylamide or agarose gel electrophoresis. Each of the processed cDNA segments is annealed into the appropriate restriction site of an expression vector, such as /gtll (Young and Davis, Proc. Natl. Acad. of Sci. 80, 1194-1198), pCQV2 (Queen, C., J. Mol. and Appl. Genetics 2:1-10, 1983), pMB9-derived vectors, (Roberts, et al. Proc. Natl. Acad. Sci. 76, 760-764, 1979), or pBR322-derived vectors (Jay et al., Proc. Natl. Acad. Sci. 78, 5543-5548, 1981). The recombinant expression vector is introduced into E. coli RR1 by the method of Peacock et al. supra. Bacterial colonies containing the preproGRF-related cDNA segments are selected by DNA hybridization with probe B or by antibody screening (Young & Davis supra.), and 50 hybridization-positive colonies containing each of the preproGRF genes are selected for further culturing. The correct structure of the putative cDNA-vector is confirmed by restriction enzyme analysis.

In order to examine bacterial colonies for production of preproGRF-related peptides directed by the bacterial expression vector in E. coli, the presence of GRF protein sequences in the colonies is determined by radioimmunoassay. Cell lysates are prepared, bound to polyvinyl chloride microtiter wells, exposed to GRF-specific antibody, and antibody-antigen complex is quantitated by further binding of $^{125}$I-labeled Protein A by the method described by Kohler, G. Y., in Hybridoma Techniques, Cold Spring Harbor Lab., Cold Spring Harbor, N.Y. 1980. Identification of GRF peptide sequences in the lysate of some of the seven preproGRF-107 colonies and some of the twelve preproGRF-108 colonies indicates that the cDNA's express their respective protein products in these colonies.

A colony of E. coli transformed with preproGRF-107-related genes and producing reasonable amounts of peptide that is reactive with an appropriate specific antibody is selected for further culturing, as is a colony of E. coli likewise transformed with preproGRF-108-related genes. Lysates are prepared from each of the colonies, and each lysate is fractionated by exclusion chromatography followed by electrophoresis on polyacrylamide gels. Exclusion chromatography fractions which contain proteins with molecular weights in the 12,000 to 13,000 range are purified by affinity chromatography on columns of Sepharose beads coupled with anti-GRF antibodies, and/or by ion-exchange chromatography, all of which purification methods are generally known in the art. Final purification may be obtained by high pressure liquid chromatography (Guillemin, et al. supra).

The quantity of the respective preproGRFs in the recovered fractions are estimated by radioimmunoassays in which GRF-reactive antiserum is first incubated with various dilutions of the fractions and then exposed to $^{125}$I-labeled synthetic GRF-44. The concentrations of preproGRF in the various dilutions is calculated by comparing the concentrations of antibody-bound and unbound labeled GRF with standard curves derived from controls, in which the GRF antiserum is initially exposed to known concentrations of unlabeled GRF and then to known concentrations of labeled GRF. The results indicate that the purified fractions each contain preproGRF in the range of about 75 to about 80 percent of the total protein.

Although neither of the two forms of unprocessed preproGRF or hybrid proteins containing bacterial sequences as well as GRF sequences have been shown to have GRF activity, these substances nevertheless represent valuable compounds. It may well prove to be the case that, if the precursors are administered to an animal, the precursor will subsequently be processed in vivo into GRF. In any case, preproGRF may be processed in vitro by exposing the preproGRF to a human hypothalamic extract containing

0134085

processing enzymes. It is likely that a hypothalamic extract from a non-human species can be found which processes human preproGRF to GRF. Either _in_ _vivo_ or _in_ _vitro_ enzymatic processing of preproGRF would be expected to amidate the terminal carboxyl group of GRF 44. This is a distinct advantage of the natural gene over a synthetic oligodeoxynucleotide which encodes only the GRF amino acid sequence without the processing sequences found in preproGRF. The processing may also proceed on partially purified fractions which are thereafter purified as described above. In the cases where a hybrid protein containing bacterial sequences at the amino-terminus is the product of bacterial expression, partial hydrolysis with a reagent, such as cyanogen bromide, may be necessary to release the preproGRF for further processing _in_ _vitro_ by the tissue extracts.

The preproGRF gene fragments engineered for expression in bacteria can also be used for expression in eukaryotic systems, either in yeast or mammalian cells. In the case of yeast, mammalian interferons were found to be properly secreted and processed when expressed using a yeast vector, YEp1PT (Hitzeman et al. _Science_, _219_, 620-625, 1983). PreproGRF may be similarly processed and secreted, which would aid in the purification process, the preproGRF may then serve as a substrate for _in_ _vitro_ processing, as described above. Alternatively, the cDNA segments found to code for preproGRF can also be introduced into a human hypothalamic cell line or a rat pituitary cell line where enzymes are available for processing preproGRF-107 and preproGRF-108. The cDNA can be cloned into such expression vector as the pSV2 family of SV40-derived vectors (_Mulligan, R. C. and Berg, P._ in _Eukaryotic_ _Viral Vectors_, Y. Gluzman, ed. Cold Spring Harbor Lab., Cold Spring Harbor, NY.) or into the newer retroviral vectors, Mulligan, P., _Science_ _209_: 1422-1427 (1980).

The cDNA can then be delivered into the hypothalamic cells by microinjection, DNA transfection, or viral infection. Cells that stably integrate the introduced cDNA can be selected by a selection protocol for either gpt or neo, the 2 dominant markers provided by the vector system. GRF production by the colonies would be assayed by the general method described above using appropriate antibodies. Several of the colonies should produce GRF at levels well above the level of GRF produced by the non-transformed cell lines. Thus, the isolated preproGRF cDNA is considered suitable for transformation into eukaryotic host cells, and the product expressed will, in at least some instances, be processed into GRF by enzymes within the host cell in the normal manner.

While the invention has been described in terms of certain preferred embodiments, modifications obvious to one with ordinary skill in the art may be made without departing from the scope of the invention. For example, it is understood that the preproGRF cDNAs described herein represent only the precise structure of two naturally occurring gene segments. It is expected that slightly modified alleles will be found encoding for similarly functioning proteins, and such gene segments and proteins are considered to be equivalents for the purpose of this invention. It is also known that slightly modified homologs of the proteins could be synthesized and that many such modified homologs would be expected to exhibit preproGRF activity, and these are likewise considered within the scope of the invention. DNA having equivalent codons is considered within the scope of the invention, as are synthetic gene segments that encode homologous peptides that exhibit preproGRF activity.

-1-    0134085

Claims for the contracting states:BE,FR,DE,IT,LU,NL,SE,CH,UK

1. DNA encoding a precursor of hpGRF which includes a nucleotide sequence that encodes the entire hpGRF peptide sequence, a peptide segment linked to the amino terminus of said hpGRF peptide and a peptide segment linked to the carboxyl-terminus of said hpGRF peptide, said terminus segments including signal segments for directing their enzymatic removal from said hpGRF peptide and for directing enzymatic amidation of the hpGRF carboxyl terminus.

2. DNA according to Claim 1 wherein said nucleotide sequence encodes for the amino acid sequence: Arg-Arg-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-Gly-Arg.

3. DNA according to Claim 1 wherein said nucleotide sequence encodes for the amino acid sequence: Met-Pro-Leu-Trp-Val-Phe-Phe-Phe-Val-Ile-Leu-Thr-Leu-Ser-Asn-Ser-Ser-His-Cys-Ser-Pro-Pro-Pro-Pro-Leu-Thr-Leu-Arg-Met-Arg-Arg-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-Gly-Arg-Gln-Val-Asp-Ser-Met-Trp-Ala-Glu-Gln-Lys-Gln-Met-Glu-Leu-Glu-Ser-Ile-Leu-Val-Ala-Leu-Leu-Gln-Lys-His-R-Arg-Asn-Ser-Gln-Gly, where R is selected from the group consisting of Ser and des-R.

4. DNA according to any of Claims 1-3 wherein said nucleotide sequence contains the following sequence: CGG CGG TAT GCA GAT GCC ATC TTC ACC AAC AGC TAC CGG AAG GTG CTG GGC CAG CTG TCC GCC CGC AAG CTG CTC CAG GAC ATC ATG AGC AGG CAG CAG GGA GAG AGC AAC CAA GAG CGA CGA GCA AGG GCA CGG CTT GGT CGT.

5. DNA according to any of Claims 1-3 wherein said nucleotide sequence contains the following sequence:     ATG CCA CTC TGG GTG TTC TTC TTT GTG ATC CTC ACC CTC AGC AAC AGC TCC CAC TGC TCC CCA CCT CCC CCT TTG ACC CTC AGG ATG CGG CGG TAT GCA GAT GCC ATC TTC ACC AAC AGC TAC CGG AAG GTG CTG GGC CAG CTG TCC GCC CGC AAG CTG CTC CAG GAC ATC ATG AGC AGG CAG CAG GGA GAG AGC AAC CAA GAG CGA CGA GCA AGG GCA CGG CTT GGT CGT CAG GTA GAC AGC ATG TGG GCA GAA CAA AAG CAA ATG GAA TTG GAG AGC ATC CTG GTG GCC CTG CTG CAG AAG CAC NNN AGG AAC TCC CAG GGA, where NNN is selected from the group consisting of AGC and des-NNN.

6. A DNA sequence according to any one of Claims 1-5 inserted within an expression vector capable, in a transformant microorganism or cell culture, of expressing the amino acid sequence encoded by said DNA sequence.

7. The expression vector according to Claim 6, inserted within a transformant microorganism or cell culture, wherein the product of said DNA sequence is expressed as a polypeptide having the encoded amino acid sequence.

8. A polypeptide expressed in the microorganism or cell culture of Claim 7 having the encoded amino acid sequence or an enzymatically processed fragment thereof.

9. A method of producing a polypeptide comprising, providing a DNA nucleotide sequence that encodes the entire hpGRF peptide sequence, a peptide segment linked to the amino terminus of said hpGRF peptide and a peptide segment linked to the carboxyl-terminus of said hpGRF peptide, said terminus segments including signal segments for directing their enzymatic removal from said hpGRF peptide and for directing enzymatic amidation of the hpGRF carboxyl terminus, linking said DNA sequence to additional DNA sequences to produce an expression vector capable, in a transformant microorganism or cell culture, of expressing a polypeptide having the amino acid sequence encoded by said DNA sequence, transforming a cell of either a microorganism or a cell culture with said expression vector, culturing said transformed cell, and obtaining the polypeptide having the amino acid sequence encoded by said DNA sequence or an enzymatically processed fragment thereof.

10.  A method according to Claim 9 wherein said nucleotide sequence encodes for the amino acid sequence:  Arg-Arg-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-Gly-Arg.

11.  A polypeptide sequence as defined in Claim 2 or Claim 3.

Claims for the contracting state: AT

1.　　A method for producing a DNA sequence encoded for an hpGRF precursor and including a nucleotide sequence encoded for the peptide, a nucleotide sequence encoded for a flanking amino acid sequence linked to the hpGRF amino terminus and a nucleotide sequence encoded for a flanking amino acid sequence linked to the hpGRF carboxy terminus, said flanking sequences including signal segments for directing their enzymatic removal and for enzymatic amidation of the carboxy terminus of the hpGRF, the method comprising isolating mRNA from a pancreatic source of hpGRF, producing the cDNA reverse transcript of said mRNA, annealing said cDNA to an expression vector, transforming the vector into E. coli cells, culturing said transformants, selecting cells transformed with cDNA encoding peptides comprising hpGRF sequences, and excising the cDNA from the vector.

2.　　A method of producing preproGRF which method comprises transforming a host cell with a DNA sequence encoded for said peptide, and culturing the transformants.

3.　　A method of producing preproGRF comprising, isolating a DNA sequence which encodes a polypeptide having the amino acid sequence: Met-Pro-Leu-Trp-Val-Phe-Phe-Phe-Val-Ile-Leu-Thr-Leu-Ser-Asn-Ser-Ser-His-Cys-Ser-Pro-Pro-Pro-Pro-Leu-Thr-Leu-Arg-Met-Arg-Arg-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-Gly-Arg-Gln-Val-Asp-Ser-Met-Trp-Ala-Glu-Gln-Lys-Gln-Met-Glu-Leu-Glu-Ser-Ile-Leu-Val-Ala-Leu-Leu-Gln-Lys-His-R-Arg-Asn-Ser-Gln-Gly, wherein R is selected from the group consisting of Ser and des-R,

transforming a host cell with said isolated DNA sequence in a manner such that said host cell expresses the encoded polypeptide, and

culturing said host cell so that preproGRF is expressed.

4. The preproGRF product obtained from the process claim 3.

5. A method according to claim 3 including exposing said preproGRF to processing enzymes which remove peptide segments from the amino-terminus and the carboxyl-terminus of the said preproGRF, leaving amino acid residues 32-75 of said preproGRF, and amidate the carboxyl-terminus.

6. A method according to claim 3 wherein said host cell is selected which has said processing enzymes which process preproGRF to GRF within said cell.

European Patent Office

**EUROPEAN SEARCH REPORT**

| DOCUMENTS CONSIDERED TO BE RELEVANT | | EP 84304581.6 |
|---|---|---|

| Category | Citation of document with indication, where appropriate, of relevant passages | Relevant to claim | CLASSIFICATION OF THE APPLICATION (Int. Cl.4) |
|---|---|---|---|
| A,P | EP - A1 - 0 108 387 (HOFFMANN-LA ROCHE)<br>* Claims 1,2,8,12; fig. 2 *<br>-- | 1,2,4,<br>6-8,11 | C 12 N 15/00<br>C 07 H 21/04<br>C 07 K 7/10<br>C 07 K 13/00<br>C 12 P 21/02 |
| D,A | SCIENCE, vol. 218, no. 4572, November 5, 1982, Washington, D.C. (USA)<br>R. GUILLEMIN et al. "Growth Hormone-Releasing Factor from a Human Pancreatic Tumor that coused Acromegaly"<br>pages 585-587<br>* Totality *<br>-- | | |
| A | EP - A1 - 0 020 147 (THE REGENTS OF THE UNIVERSITY OF CALIFORNIA)<br>---- | | |

TECHNICAL FIELDS SEARCHED (Int. Cl.4)

C 12 N
C 07 H
C 07 K
C 12 P

The present search report has been drawn up for all claims

| Place of search | Date of completion of the search | Examiner |
|---|---|---|
| VIENNA | 08-10-1984 | FARNIOK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82